# EUROPÄISCHE PATENTSCHRIFT

(11) **EP 1 322 278 B1**
(45) Veröffentlichungstag und Bekanntmachung des Hinweises auf die Patenterteilung: **13.08.2008**
(21) Anmeldenummer: 01982372.3
(22) Anmeldetag: 28.09.2001
(51) Int. Cl.: A61K 6/06, C25D 1/14, A61C 5/08, C04B 35/119, C04B 35/622

(54) **VERFAHREN ZUR HERSTELLUNG VON KERAMISCHEN FORMTEILEN**
METHOD FOR PRODUCING CERAMIC MOULDED PARTS
PROCEDE DE PRODUCTION DE PIECES MOULEES EN CERAMIQUE

(30) Priorität: 06.10.2000 DE 10049974
(43) Veröffentlichungstag der Anmeldung: 02.07.2003
(73) Patentinhaber: Wieland Dental + Technik GmbH & Co. KG, 75179 Pforzheim (DE)
(72) Erfinder: LAUBERSHEIMER, Jürgen, 76307 Karlsbad (DE)
(74) Vertreter: Patentanwälte Ruff, Wilhelm, Beier, Dauster & Partner
(86) Internationale Anmeldenummer: PCT/EP2001/011219
(87) Internationale Veröffentlichungsnummer: WO 2002/030361

(56) Entgegenhaltungen:
- WO-A-98/48084
- WO-A-99/50480
- US-A- 4 204 931
- US-A- 5 415 748
- N. CLAUSSEN E.A.: "Tailoring of Reaction-Bonded Al2O3 (RBAO) Ceramics" CERAMIC ENGINEERING AND SCIENCE PROCEEDINGS, Bd. 11, Nr. 7-8, 1990, Seiten 806-820, XP002187987 in der Anmeldung erwähnt

## Beschreibung

Die Erfindung betrifft ein Verfahren zur Herstellung von keramischen Formteilen für die dentale Anwendung unter Verwendung einer mindestens ein keramisches Pulver enthaltenen Mischung und Verfestigung der Mischung durch Sintern gemäß dem Oberbegriff von Anspruch 1.

Schon immer war Keramik oder "Porzellan" ein attraktiver Werkstoff, um Zähne mit sehr zahnähnlichem Aussehen in Form und Farbe nachzubilden. Seit wissenschaftlich belegt ist, daß Keramik ein chemisch beständiger, korrosionsfester und biokompatibler Werkstoff ist, der zudem noch in schier unendlicher Menge in mineralischer Form verfügbar und mit zahntechnischen Mitteln individueller, paßgenauer Zahnersatz einfach und reproduzierbar herzustellen ist, ist der Durch bruch des Werkstoffes "Dentalkeramik" eingetreten.

Um die einzige Schwäche dieses Werkstoffes, die Bruchempfindlichkeit, zu umgehen, wird zahntechnisch gefertigter Zahnersatz in der Regel schon seit Jahrzehnten als klassischer Werkstoff-Verbund hergestellt, z. B. als sogenannte Metallkeramik. Eine metallkeramische Krone oder Brücke besteht aus einem metallischem Gerüst bzw. Unterbau und einer der Zahnform nachempfundenen sogenannten Verblendung aus Dentalkeramik. Der Unterbau wird beim Einsetzen des Zahnersatzes direkt auf dem nach der zahnärztlichen Präparation verbleibenden Restzahn befestigt und wird oft als (Schutz-)Käppchen bezeichnet. Je nachdem, aus welchem Metall bzw. aus welcher Legierung die Käppchen bestehen und je nach Herstellverfahren (Gießen, Galvanoforming-Verfahren) können Probleme in Form von Korrosion und daraus resultierenden Verfärbungen, Körperunverträglichkeiten und anderes mehr entstehen, weshalb in den letzten Jahren zunehmend Systeme entwickelt werden, um vergleichbare Unterkonstruktionen aus keramischen Materialien herstellen und zahntechnisch weiterverarbeiten zu können.

Es gibt bereits mehrere funktionierende Systeme auf dem Dentalmarkt, bei denen die Keramik-Käppchen beispielsweise durch manuelles Auftragen eines Schlickers auf einen Modellstumpf, anschließendem Sinterbrand sowie nachfolgender Infiltration mit Spezialglas, durch einen Pressvorgang unter Temperatureinwirkung bzw. mehrere Systeme, bei denen die Käppchen aus vorgesintertem Keramikblöcken digital gefräst werden, hergestellt werden. Allen diesen sogenannten Vollkeramik-Systemen ist jedoch gemeinsam, daß die Paßgenauigkeit metallischer Käppchen auf dem Restzahn, ob sie nun gegossen sind oder durch galvanische Prozesse entstehen, in der Regel nicht erreicht werden. Beispielsweise muß beim digitalen Ausfräsen der Käppchen nach einem digital aufgenommenen Datensatz aus festem Material spanabhebend gefräst werden. Das Scannen des Zahnstumpfes und das Fräsen bedingen, je nach der digitalen Auflösung der Systemkomponenten bereits Ungenauigkeiten. Zudem sind die Systeme in der Anschaffung meist sehr teuer.

Es ist bekannt, dentale Formteile elektrophoretisch abzuscheiden. Die elektrophoretische Formgebung erzeugt aus dispergierten, frei beweglichen Teilen einen Gegenstand mit definierter geometrischer Form unter Ausnutzung der Kraftwirkung eines elektrischen Feldes auf diese Teilchen infolge deren elektrischer Ladung. Dabei ist die Masse des elektrophoretisch abgeschiedenen Materials proportional der angelegten Spannung.

In der WO 99/50480 ist ein Verfahren zur elektrophoretischen Abscheidung von keramischen Partikeln zur Herstellung von Keramikkörpern für dentale Anwendungen beschrieben. Dabei wird zunächst eine Suspension von keramischen Partikeln in einem polaren Lösungsmittel, insbesondere in einem Alkohol, hergestellt. Die Suspension besteht zu mindestens 5 Gew. % aus keramischen Partikeln. Danach wird ein Strom durch die Suspension geleitet, der die Abscheidung der keramischen Partikeln an einer als dentaler Formkörper ausgebildeten Elektrode bewirkt. Alternativ kann auch eine Suspension aus Keramik- und Glaspartikeln abgeschieden werden.

Ferner ist es bekannt, Aluminiumoxidkörper mit geringem Schrumpf beim Sintern herzustellen, indem als Ausgangsmaterial eine Mischung von Aluminiumpartikeln und Aluminiumoxidpulverpartikeln eingesetzt wird. Durch die Oxidation der Aluminiumpartikel zu Aluminiumoxid wird der Sinterschrumpf kompensiert (tailoring of reaction-bonded Al₂O₃ (RBAO) ceramics, Ceram. Eng. Sci. Proc. vol. 11 pp. 806-820 (1990)). In der Zahntechnik müssen außer geringem Schrumpf aber zahlreiche andere Kriterien, wie Bruchfestigkeit, Porenfreiheit, Härte und Farbe aufeinander abgestimmt werden.

### Aufgabe und Lösung

Aufgabe der Erfindung ist es, ein Verfahren zur Herstellung von keramischen Formteilen für dentale Anwendungen bzw. ein dentales Formteil zu schaffen, das gegenüber dem Stand in der Dentaltechnik verbesserte Eigenschaften aufweist. Zudem soll der beim Sintern des dentalen Formteils auftretende Schrumpf verringert werden.

Diese Aufgabe wird durch ein Verfahren zur Herstellung eines keramischen Formteiles mit den Merkmalen des Anspruches 1, sowie durch ein dentales Formteil mit den Merkmalen des Anspruches 15 gelöst. Weiterbildungen der Erfindung sind in den abhängigen Ansprüchen dargestellt.

Das erfindungsgemäße Verfahren zur Herstellung des keramischen Formteiles zeichnet sich dadurch aus, daß in der Mischung auch mindestens ein metallisches Pulver enthalten ist und das Sintern unter oxidativen Bedingungen durchgeführt wird. Dabei liegen das mindestens eine keramische Pulver und das mindestens eine metallische Pulver als dispergierte Partikel in ein flüssiges Dispersionsmittel enthaltenen Dispersion vor und werden durch Elektrophorese auf einem dentalen Grundkörper abgeschieden. Die Kombination von elektrophoretischer Abscheidung in Verbindung mit der speziellen Mischung ergibt unerwartet gute Ergebnisse.

Beim Sintern von Keramiken kommt es zu einem Volumenschrumpf, der in der Regel im Bereich von 15 % bis 25 % des ursprünglichen Volumens liegt. Der Sinterschrumpf kann zu Rissen im Gefüge oder gar dem Brechen des Formteiles führen. Um diesem Sinterschrumpf entgegenzuwirken, ist in der Mischung, die zur Herstellung des keramischen Formteiles verwendet wird, mindestens ein metallisches Pulver beigemischt. Das Metall oxidiert dann in einem Oxidationsschritt durch Reaktion mit Sauerstoff und expandiert dabei. Der Oxidationsschritt kann in einem Brennschritt zusammen mit dem Sintern durchgeführt werden. Zunächst erfolgt die Oxidation, danach das Sintern. Der resultierende Sinterkörper zeigt ein homogenes oxidisches Gefüge. Bei entsprechenden Mengenverhältnissen von eingesetztem Metall zu Oxidpulver kann diese durch die Oxidation des Metalls bedingte Expansion durch den Sinterschrumpf des keramischen Formkörpers gerade kompensieren.

Das erfindungsgemäße Verfahren eignet sich zur Herstellung von allen denkbaren keramischen Form- oder Gerüstteilen in der Dentaltechnik, wie Kronen, Brücken oder dergleichen. Eine keramische Krone beispielsweise besteht aus einem Unterbau und einer der Zahnform nachempfundenen sogenannten Verblendung aus Dentalkeramik. Der Unterbau wird beim Einsetzen des Zahnersatzes direkt auf dem nach der zahnärztlichen Präparation verbleibenden Restzahn befestigt und wird oft als Käppchen bezeichnet. Mit dem erfindungsgemäßen Verfahren ist es möglich, solche Käppchen herzustellen, die eine hohe Paßgenauigkeit beim Aufsetzen auf den verbleibenden Restzahn aufweisen.

Bei der elektrophoretischen Abscheidung ist es notwendig, zunächst einen keramischen Schlicker herzustellen. Unter keramischen Schlicker versteht man Suspensionen dispergierter keramischer und/oder metallischer Pulver in geeigneten flüssigen Dispersionsmitteln. Als Dispersionsmittel werden vorzugsweise polare Dispersionsmittel eingesetzt. Bevorzugtes Dispersionsmittel hierbei ist Wasser. Aber auch Alkohole, bevorzugt niedere Alkohole, sind als Dispersionsmittel geeignet.

Der keramische Schlicker kann an einer der Form eines dentalen Grundkörpers entsprechenden Elektrode elektrophoretisch abgeschieden werden. Als dentaler Grundkörper kann ein Duplikat eines präparierten Zahnstumpfes eingesetzt werden. In den meisten Fällen wird bei der Elektrophorese eine anodische Abscheidung der dispergierten Feststoffpartikel beobachtet. Die Keramikpartikel werden also negativ aufgeladen und wandern zum Pluspol, also zur vom dentalen Grundkörper gebildeten Anode. Es ist jedoch auch eine kathodische Abscheidung der Feststoffpartikel möglich.

Der Anteil des metallischen Pulvers an der Gesamtmenge der dispergierten Partikel kann in großen Bereichen schwanken. Er beträgt beispielsweise ca. 10 Gew. % bis 80 Gew. %, vorzugsweise 30 Gew. % bis 60 Gew. %. Der dispergierte Anteil im keramischen Schlicker besteht zum Teil, vorzugsweise etwa zu einem Drittel, aus metallischem Pulver. Der Rest wird von keramischen Pulverpartikeln gebildet, die im folgenden Primärpartikel genannt werden. Die Primärpartikel besitzen einen mittleren Durchmesser, der im Bereich von 0,4 µm bis 0,8 µm, vorzugsweise 0,6 µm bis 0,7 µm liegt. Der mittlere Durchmesser der metallischen Pulverpartikel kann deutlich größer sein. Vorzugsweise liegt er im Bereich von 1 µm bis 50 µm, vorzugsweise 2 µm bis 10 µm.

Um eine Oxidation des metallischen Pulvers bei dem oxidativen Sintervorgang zu gewährleisten, handelt es sich beim metallischen Pulver vorzugsweise um ein Pulver aus Nicht-Edelmetall. Dabei kann es sich bei dem Metallpulver um ein Metall handeln, dessen Metallionen im keramischen Pulver enthalten sind. Nach dem Sintervorgang, bei dem das Metallpulver zu Metalloxid oxidiert wird, sind die nunmehr gebildeten Metalloxidpartikel praktisch nicht mehr von den keramischen Primärpartikeln unterscheidbar. Der dentale Formkörper besteht also aus einem homogenen oxidischen Gefüge. Als Metallpulver kann beispielsweise Aluminium- und/oder Zirkoniumpulver eingesetzt werden.

Aus dem keramischen Pulver und dem metallischen Pulver kann eine im wesentlichen homogene Mischung hergestellt werden. Die Mischung kann z. B. durch Mischmalen der beiden Pulver im festen Zustand hergestellt werden. Dabei kann der mittlere Durchmesser der metallischen Pulverpartikel verkleinert werden.

Bei der Herstellung des keramischen Schlickers aus keramischen Primärpartikeln und metallischem Pulver können verschiedene Hilfsstoffe zugegeben werden. Zudem kann der keramische Schlicker oder einzelne Bestandteile des Schlickers einer Vorbehandlung unterzogen werden. Der bevorzugte pH-Wert für die elektrophoretische Abscheidung liegt im Bereich von pH 5 bis 9. Gegebenenfalls kann der pH-Wert durch Zugabe einer Säure oder Base, beispielsweise Citronensäure oder Natriumpyrophosphat, eingestellt werden. Um das Dispergieren der keramischen und metallischen Pulverpartikel im Dispersionsmittel zu verbessern, kann ein Dispergierhilfsmittel zugegeben werden. Als Dispergierhilfsmittel kann beispielsweise Natriumpyrophosphat dienen. Als keramische Primärpartikel oder keramisches Pulver kann eine Mischung aus Partikeln verschiedener Oxidkeramiken eingesetzt werden, die im festen Zustand vorgemischt werden. Beispielsweise können die Primärpartikel aus einer Feststoffmischung aus Aluminiumoxidpartikeln und Zirkoniumoxidpartikeln im Verhältnis 3:1 hergestellt werden. Danach können die metallischen Pulverpartikel zugegeben und gemeinsam mit den keramischen Primärpartikeln mischgemahlen werden. Die Mischung aus keramischen und metallischen Pulverpartikeln kann unter Rühren in das Dispersionsmittel gegeben werden, und um das Dispergieren weiter zu verbessern, mit Ultraschall behandelt werden. Dem dabei entstandenen keramischen Schlicker kann ein organisches Additiv, beispielsweise ein mehrwertiger Alkohol, insbesondere Polyvinylalkohol, zugegeben werden, um beim Abscheiden eine bessere Agglomeration der Partikel zu erreichen. Um das Trocknen des abgeschiedenen keramischen Formteiles zu erleichtem, kann dem keramischen Schlicker ein Trocknungshilfsmittel zugegeben werden. Das Trocknungshilfsmittel kann ein Amid sein, beispielsweise Formamid.

Nach der Herstellung des Schlickers erfolgt dessen elektrophoretische Abscheidung an einer der Form eines dentalen Grundkörpers entsprechenden Elektrode. Bei der Elektrophorese wandern die dispergierten keramischen und metallischen Pulverpartikel unter dem Einfluß eines elektrischen Feldes in eine bestimmte Richtung, die von ihrer Ladung bzw. Oberflächenladung abhängt. Der keramische Schlicker wird also an der Elektrode, vorzugsweise anodisch, abgeschieden. Grundsätzlich gibt es zwei Arten, die elektrophoretische Abscheidung durchzuführen. Sie kann bei konstanter Stromstärke und daraus resultierend zunehmender Spannung durchgeführt werden. Es ist aber auch möglich, bei konstanter Spannung und daraus resultierend abnehmender Stromstärke zu arbeiten. Bei konstanter Stromstärke wird diese aus einem Bereich von 1 mA bis 100 mA, vorzugsweise 10 mA bis 50 mA gewählt. Die Anfangsspannung liegt dabei im Bereich von 0,5 V bis 30 V, vorzugsweise 1 V bis 5 V. Bei konstanter Spannung wird diese aus einem Bereich von 1 V bis 100 V, vorzugsweise 2 V bis 10 V gewählt. Dabei beträgt die Anfangsstromstärke ca. 1 mA bis 100 mA, vorzugsweise 10 mA bis 50 mA.

Nach der elektrophoretischen Abscheidung kann das abgeschiedene Formteil nachbehandelt werden. Das abgeschiedene Formteil, das vor dem Brennen auch Keramikgrünling genannt wird, wird vorzugsweise zunächst getrocknet, beispielsweise mittels einer Mikrowelle. Die Keramikgrünlinge haben eine Gründichte von etwa 30 % bis 70 %, typischerweise etwa 50 % der theoretischen Dichte des keramischen Formteils.

Das Brennen des dentalen Formteils kann in einem kombinierten Oxidations- und Sinterprozeß durchgeführt werden. Zunächst erfolgt bei niedriger Temperatur der Oxidationsschritt, bei dem das metallische Pulver zu Metalloxid oxidiert wird. Danach erfolgt bei höherer Temperatur das Sintern. Dabei kann die Dichte des dentalen Formteils auf über 90 % der theoretischen Dichte der Keramik erhöht werden. Je nach Zusammensetzung und Gehalt der dispergierten Pulver in den zur Abformung verwendeten Schlicker können die Grünlinge bei Temperaturen zwischen 700° und 1600°, insbesondere 900° und 1400° gebrannt werden. Die Dauer des Sintervorganges kann mehrere Stunden, beispielsweise 2 bis 8, typischerweise ca. 5 Stunden lang dauern. Die Aufheizung auf die Sintertemperatur erfolgt vorzugsweise langsam, beispielsweise in einem Schritt von 1° bis 20° pro Minute, insbesondere 5° bis 10° pro Minute.

Nach dem Sintern kann das gebrannte dentale Formteil mit Glaspartikeln infiltriert werden, um die beim Sintern entstandenen Poren im Gefüge zu verschließen.

Weiter ist ein dentales Formteil beschrieben, das mit Hilfe des beschriebenen Verfahrens herstellbar ist.

Der Anteil der oxidierten metallischen Partikel im Gefüge des dentalen Formteiles liegt wie im keramischen Schlicker bei ca. 10 Gew. % bis 80 Gew. %, vorzugsweise 30 Gew. % bis 60 Gew. %. Der mittlere Durchmesser der oxidierten metallischen Partikel im Gefüge liegt vorzugsweise im Bereich von 1 µm bis 10 µm, vorzugsweise 2 µm bis 5 µm.

Wie bereits erwähnt, kann das dentale Formteil, dessen Gefüge keramische Primärpartikel und oxidierte metallische Partikel enthält, als kappenartiger Hohlformkörper ausgebildet sein. Er kann beispielsweise als Unterbau bzw. Käppchen einer keramischen Krone ausgebildet sein. Die Wandstärke des dentalen Formteiles kann 0,1 mm bis 1 mm, vorzugsweise 0,3 mm bis 0,7 mm betragen.

Die beschriebenen Merkmale und weitere Merkmale der Erfindung ergeben sich aus der nachfolgenden Beschreibung eines Beispiels in Verbindung mit den Unteransprüchen. Hierbei können die einzelnen Merkmale jeweils für sich oder in Kombination miteinander verwirklicht sein.

### Beispiel

Das erfindungsgemäße Verfahren zur Herstellung von keramischen Formteilen sowie das dentale Formteil soll beispielhaft anhand der Herstellung einer keramischen Krone erläutert werden.

### Herstellung des dentalen Grundkörpers (Elektrode)

Zunächst wird der dentale Grundkörper hergestellt, der bei der elektrophoretischen Abscheidung als Elektrode dient. Der dentale Grundkörper ist im beschriebenen Beispiel ein Duplikatstumpf eines Zahnes, der elektrisch z. B. mit Leitsilberlack kontaktiert ist und als Elektrode in einem Stromkreis geschaltet ist. Zur Herstellung des Duplikatstumpfes wird zunächst ein Negativabdruck des Zahnes, beispielsweise mit einer Silikonmasse, angefertigt. Mit Hilfe dieses Negativabdruckes kann der Duplikatstumpf angefertigt werden, beispielsweise gegossen werden. Als Stumpfmaterial werden dabei bei den Brenntemperaturen stabile Materialien, bevorzugt in der Zahntechnik gebräuchliche sogenannte feuerfeste Einbettmassen gewählt, wie sie beispielsweise in der dentalen Gußtechnik verwendet werden, so z. B. gipsgebundene Einbettmassen oder Löteinbettmassen.

### Schlickerherstellung mit keramischen Primärpartikeln und metallischen Partikeln:

100 g einer Mischung der Al₂O₃ - und ZrO₂ - Pulver (Gew.-Verhältnis 3:1) und 50 g Aluminium-Pulvers werden 2 h mischgemahlen und getrocknet. Dann werden zunächst zu 250 ml Wasser je 0,5 g Natriumpyrophosphat und Citronensäure vorgegeben und gerührt, anschließend die o. a. Pulvermischung portionsweise zugegeben und intensiv gerührt. Danach wird die Dispersion einer Ultraschallbehandlung unterzogen. Anschließend werden der Dispersion 20 ml der 5%igen PVA-Lösung, MG 72.000 langsam eingerührt, nach einer Zeit noch 6 ml Formamid als DCCA (= Trocknungshilfsmittel) zugegeben und dann über Nacht intensiv gerührt.
Verwendete Chemikalien: Aluminiumoxidpulver CT 3000 SG, Fa. ALCOA; Zirkonoxidpulver SC 15, MEL CHEMICALS; Aluminium-Pulver APS, 3-4.5 micron, Fa. ALFA; Citronensäure-Monohydrat 99,5 %, Fa. MERCK; Natrium-pyrophosphat-Dekahydrat, Fa. RIEDEL DE HAEN; Polyvinylalkohol, Molekulargewicht 72.000, Fa. FLUKA.

### Elektrophoretische Abscheidung

In ein 100 ml-Laborbecherglas werden etwa 70 ml der oben beschriebenen Schlicker eingefüllt und etwa 5 min. einer Ultraschallbehandlung unterzogen. Dann wird, unter langsamen Rühren (ca. 100 U/min.) mit Hilfe einer Gleichstromquelle, mit einem Platinblech als Kathode und einem mit Leitsilberlack kontaktierten Modell-Zahnstumpf aus feuerfester Stumpfmasse (L 36, Fa. HINRICHS) als Anode bei typischen Spannungen von 1 - 5 V und Strömen von 20 - 100 mA während einer Prozeßdauer von typischerweise etwa 10 - 15 Minuten die keramischen Bestandteile des Schlickers in Form einer glatten, optisch dichten, etwa 0,4 mm bis 0,5 mm dicken Schicht abgeschieden.

### Sintervorgang

Wurden die Käppchen aus einem Schlicker abgeschieden, in dem eine Mischung aus Metall- und oxidkeramischem Pulver suspendiert sind, wird mit einem zusätzlichen Oxidationsschritt gearbeitet. Der Sinterschritt ist hier ein Reaktionssinter-Prozeß:
- Aufheizrate bis 900°C: 7,5°C/min
- Haltezeit 2 Stunden bei 950°C
- Aufheizrate bis 1150°C: 7,5°C/min
- Haltezeit 4 h bei 1150°C, dann im Ofen abkühlen

## Patentansprüche

1. Verfahren zur Herstellung von keramischen Formteilen für die dentale Anwendung unter Verwendung einer mindestens ein keramisches Pulver enthaltenen Mischung und Verfestigen der Mischung durch Sintern, wobei in der Mischung auch mindestens ein metallisches Pulver enthalten ist und das Sintern unter oxidativen Bedingungen durchgeführt wird, **dadurch gekennzeichnet, dass** das mindestens eine keramische Pulver und das mindestens eine metallische Pulver als dispergierte Partikel in einer ein flüssiges Dispersionsmittel enthaltenen Dispersion vorliegen und aus dieser durch Elektrophorese auf einem dentalen Grundkörper abgeschieden werden.

2. Verfahren nach Anspruch 1, **dadurch gekennzeichnet, daß** der Anteil an metallischem Pulver an der Gesamtmenge der dispergierten Partikel zwischen 10 Gew. % und 80 Gew. %, vorzugsweise zwischen 30 Gew. % und 60 Gew. % beträgt.

3. Verfahren nach Anspruch 1 oder 2, **dadurch gekennzeichnet, daß** der mittlere Durchmesser des Metallpulvers im Bereich von 1 µm bis 50 µm, vorzugsweise 2 µm bis 10 µm liegt.

4. Verfahren nach einem der vorhergehenden Ansprüche, **dadurch gekennzeichnet, daß** es sich bei dem metallischen Pulver um ein Pulver aus Nicht-Edelmetall handelt.

5. Verfahren nach einem der vorhergehenden Ansprüche, **dadurch gekennzeichnet, daß** es sich bei dem Metallpulver um ein Metall handelt, dessen Metallionen im keramischen Pulver enthalten sind.

6. Verfahren nach einem der vorhergehenden Ansprüche, **dadurch gekennzeichnet, daß** es sich bei dem Metallpulver um Aluminium- und/oder Zirkoniumpulver handelt.

7. Verfahren nach einem der vorhergehenden Ansprüche, **dadurch gekennzeichnet, daß** eine im wesentlichen homogene Mischung aus keramischem Pulver und metallischem Pulver hergestellt wird, vorzugsweise durch Mischmahlen der beiden Pulver.

8. Verfahren nach einem der vorhergehenden Ansprüche, **dadurch gekennzeichnet, daß** es sich bei dem flüssigen Dispersionsmittel um mindestens ein polares Dispersionsmittel, insbesondere um Wasser handelt.

9. Verfahren nach einem der vorhergehenden Ansprüche, **dadurch gekennzeichnet, daß** die Dispersion mindestens ein organisches Additiv, vorzugsweise mindestens einen mehrwertigen Alkohol, insbesondere Polyvinylalkohol enthält.

10. Verfahren nach einem der vorhergehenden Ansprüche, **dadurch gekennzeichnet, daß** die Dispersion mindestens ein Trocknungshilfsmittel, vorzugsweise mindestens ein Amid, insbesondere Formamid enthält.

11. Verfahren nach einem der vorhergehenden Ansprüche, **dadurch gekennzeichnet, daß** die Stromstärke bzw. Spannung aus einem Bereich von 1 mA bis 100 mA bzw. 1 V bis 100 V, vorzugsweise 10 mA bis 50 mA bzw. 2 V bis 10 V gewählt wird.

12. Verfahren nach Anspruch 11, **dadurch gekennzeichnet, daß** die Anfangsspannung bzw. Anfangsstromstärke von 0,5 V bis 30 V bzw. 1 mA bis 100 mA, vorzugsweise 1 V bis 5 V bzw. 10 mA bis 50 mA beträgt.

13. Verfahren nach einem der vorhergehenden Ansprüche, **dadurch gekennzeichnet, daß** die Sintertemperatur im Bereich von 700 °C bis 1600 °C, insbesondere 900°C bis 1400 °C gehalten wird.

## Claims

1. Process for producing shaped ceramic parts for dental applications, using a mixture containing at least one ceramic powder and consolidating the mixture by sintering, with at least one metallic powder also being present in the mixture and sintering being carried out under oxidative conditions, **characterized in that** the at least one ceramic powder and the at least one metallic powder are present as dispersed particles in a dispersion containing a liquid dispersion medium and are deposited from this onto a dental base body by electrophoresis.

2. Process according to Claim 1, **characterized in that** the proportion of metallic powder based on the total amount of dispersed particles is from 10% by weight to 80% by weight, preferably from 30% by weight to 60% by weight.

3. Process according to Claim 1 or 2, **characterized in that** the average diameter of the metal powder is in the range from 1 µm to 50 µm, preferably from 2 µm to 10 µm.

4. Process according to any of the preceding claims, **characterized in that** the metallic powder is a powder composed of base metal.

5. Process according to any of the preceding claims,
**characterized in that** the metal powder is a metal whose metal ions are present in the ceramic powder.

6. Process according to any of the preceding claims, **characterized in that** the metal powder is aluminium and/or zirconium powder.

7. Process according to any of the preceding claims, **characterized in that** an essentially homogeneous mixture of ceramic powder and metallic powder is produced, preferably by mix-milling the two powders.

8. Process according to any of the preceding claims, **characterized in that** the liquid dispersion medium is at least one polar dispersion medium, in particular water.

9. Process according to any of the preceding claims, **characterized in that** the dispersion contains at least one organic additive, preferably at least one polyhydric alcohol, in particular polyvinyl alcohol.

10. Process according to any of the preceding claims, **characterized in that** the dispersion contains at least one drying aid, preferably at least one amide, in particular formamide.

11. Process according to any of the preceding claims, **characterized in that** the current or voltage is selected from a range from 1 mA to 100 mA or from 1 V to 100 V, preferably from 10 mA to 50 mA or from 2 V to 10 V.

12. Process according to Claim 11, **characterized in that** the initial voltage or initial current is from 0.5 V to 30 V or from 1 mA to 100 mA, preferably from 1 V to 5 V or from 10 mA to 50 mA.

13. Process according to any of the preceding claims, **characterized in that** the sintering temperature is kept in the range from 700°C to 1600°C, in particular from 900°C to 1400°C.

## Revendications

1. Procédé de fabrication de pièces moulées en céramique pour applications dentaires, par recours à un mélange qui contient au moins une poudre céramique et solidification du mélange par frittage, le mélange contenant également au moins une poudre métallique et le frittage étant réalisé dans des conditions oxydantes,
**caractérisé en ce que**
la ou les poudres céramiques et la ou les poudres métalliques se présentent sous la forme de particules dispersées dans une dispersion qui contient un agent liquide de dispersion et sont déposées en suspension par électrophorèse sur un corps dentaire de base.

2. Procédé selon la revendication 1, **caractérisé en ce que** la proportion de poudre métallique par rapport à la quantité totale de particules dispersées est comprise entre 10 % en poids et 80 % en poids et de préférence entre 30 % en poids et 60 % en poids.

3. Procédé selon les revendications 1 ou 2,
**caractérisé en ce que** le diamètre moyen de la poudre métallique est compris dans la plage de 1 µm à 50 µm et de préférence de 2 µm à 10 µm.

4. Procédé selon l'une des revendications précédentes, **caractérisé en ce que** la poudre métallique est une poudre de métal non précieux.

5. Procédé selon l'une des revendications précédentes, **caractérisé en ce que** la poudre métallique est constituée d'un métal dont les ions métalliques sont contenus dans la poudre céramique.

6. Procédé selon l'une des revendications précédentes, **caractérisé en ce que** la poudre métallique est une poudre d'aluminium et/ou de zirconium.

7. Procédé selon l'une des revendications précédentes, **caractérisé en ce que** l'on forme un mélange essentiellement homogène de poudre céramique et de poudre métallique, de préférence en broyant les deux poudres lors de leur mélange.

8. Procédé selon l'une des revendications précédentes, **caractérisé en ce que** l'agent fluide de dispersion est au moins un agent de dispersion polaire et en particulier l'eau.

9. Procédé selon l'une des revendications précédentes, **caractérisé en ce que** la dispersion contient au moins un additif organique, de préférence au moins un alcool polyfonctionnel et en particulier un poly(alcool vinylique).

10. Procédé selon l'une des revendications précédentes, **caractérisé en ce que** la dispersion contient au moins un adjuvant de séchage, de préférence au moins un amide et en particulier le formamide.

11. Procédé selon l'une des revendications précédentes, **caractérisé en ce que** l'intensité du courant et la tension sont sélectionnées respectivement dans les plages de 1 mA à 100 mA et de 1 V à 100 V et de préférence de 10 mA à 50 mA et de 2 V à 10 V.

12. Procédé selon la revendication 11, **caractérisé en ce que** la tension initiale et l'intensité initiale de courant sont comprises respectivement entre 0,5 V et 30 V et entre 1 mA et 100 mA et de préférence entre 1 V et 5 V et entre 10 mA et 50 mA.

13. Procédé selon l'une des revendications précédentes, **caractérisé en ce que** la température de frittage est maintenue dans la plage de 700°C à 1 600°C, en particulier de 900°C à 1 400°C.
